# EUROPEAN PATENT SPECIFICATION

(11) **EP 0 282 512 B1**
(45) Date of publication and mention of the grant of the patent: **09.06.1999**
(21) Application number: 87905533.3
(22) Date of filing: 03.09.1987
(51) Int. Cl.: C12P 21/00, C12N 5/00, C12N 1/00

(54) **MESSENGER RNA STABILIZATION IN ANIMAL CELLS**
STABILISIERUNG VON MESSENGER-RNS IN TIERISCHEN ZELLEN
STABILISATION DE L'ARN MESSAGER DANS DES CELLULES ANIMALES

(30) Priority: 12.09.1986 US 907067
(43) Date of publication of application: 21.09.1988
(73) Proprietor: DAMON BIOTECH, INC., Needham Heights Massachusetts 02194 (US)
(72) Inventor: GILLIES, Stephen, D., Scituate, MA 02066 (US)
(74) Representative: Kirkham, Nicholas Andrew
(86) International application number: US8701964
(87) International publication number: WO8802029

(56) References cited:
- EP-A- 173 552
- EP-A- 234 157
- EP-A- 0 077 689
- WO-A-86/05807
- US-A- 4 624 926
- US-A- 4 663 281
- US-A- 4 666 848
- US-A- 4 675 285
- Journal Molecular Biology, Volume 177 issued 1964, July (London, U.K.) (K. ZARET et al) "Mutationally Altered 3' Ends of Yeast Yeast CYC1 mRNA Affect Transcript Stability and Translational Efficiency", see pages 107-135, see particularly pages 107-109, 129 and 130.
- Cell, Volume 33, issued 1983, July (Cambridge, Massachusetts, U.S.A.) (S.GILLIES et al), "A Tissue-Specific Transcription Enhancer Element is Located in the Major Intron of a Rearranged Immunoglobulin Heavy Chain Gene", see pages 717-728, se particularly pages 717, 718 and 725.
- Proceedings National Academy of Sciences U.S.A., Volume 80, issued 1983 February, (Washington, D.C., U.S.A.), (V.T. OI et al), "Immunoglobulin Gene Expression in Transformed Lymphoid Cells", see pages 825-829, see particularly pages 825, 828, and 829.
- Nature, Volume 301, issued 1983, January, (London, U.K.), (D. PENNICA et al), "Cloning and Expression of Human Tissue-Type Plasminogen Activator of cDNA in E. Coli". See pages 214-221, see particularly pages 214, 219 and 220.
- Nature, Volume 263, issued 1976, September, (London, U.K.), (N. PROUDFOOT et al) "3' Non-Coding Region Sequences in Eukaryotic Messenger RNA". See pages 211-214. See Entire Document.
- Journal Molecular Biology, Volume 167, issued 1983, July (London, U.K.) (J. ROSS et al) "Human Beta and Delta Globin Messenger RNAs Turn Over at Different Rates", see pages 607-617, see particularly pages 613 and 615.

## Description

### Background of the Invention

This invention relates to products and methods for increasing production of proteins in mammalian cells of lymphoid origin. More particularly, it relates to products and methods for increasing protein production by increasing the steady state level of translatably competent mRNA by decreasing the rate of intracellular degradation of the mRNA.

Protein production in most animal cells includes synthesis of enzymes, structural proteins, surface proteins, and numerous proteins having specialized functions such as lymphokines and hormones. Typically, relatively modest amounts of these proteins are produced. There are, however, types of animal cells which are capable of producing and secreting large amounts of proteins for systemic use in the animal body. Examples of the latter type of cells include cells of the circulatory system which produce globulins and fibrinogen, liver cells which produce serum albumin, and the beta cells of Islets of Langerhans which produce insulin. If the genetic mechanisms responsible for such high level expression could be exploited to produce lymphokines, antibodies, or other proteinaceous materials of interest, large supplies of valuable proteins could be made available.

Expression of endogenous DNA in eucaryotic cells involves transcription of the DNA into mRNA, subsequent migration of the mRNA to ribosomes followed by tRNA-mediated translation of the mRNA into proteins comprising the sequence of amino acids encoded by the mRNA between its start and stop signal codons.

As disclosed by Gillies et al in Cell, Vol. 33, pp. 717-728, July, 1983, and in co-pending U.S. application Serial No. 592,231, filed March 22, 1984, cellular enhancer elements play an important role in the high level expression of protein in specialized cells which produce large amounts of immunoglobulin. The enhancers appear to function by increasing the rate of DNA transcription into mRNA by endogenous mRNA polymerase. Increased concentrations of mRNA result in significant increases in the level of expression in such cells. The activity of such enhancer elements is independent of orientation and can be observed even when the sequence comprising the enhancer is located 10,000 base pairs or more away from the promoter for the gene encoding the protein. These cellular enhancers appear to be tissue specific, i.e., the activity of a cellular enhancer which functions in the endogenous genome of a lymphoid cell to increase production of a particular mRNA is greatly decreased or absent if the enhancer is incorporated in a vector used to transform non-lymphoid cells. However, a vector including the enhancer element, promotor, and a recombined gene encoding a desired protein, if transfected into a cell of the same type as that in which the enhancer naturally increases the transcription rate, can successfully transform the cell to express the recombined gene at high levels.

Eucaryotic DNA's comprise a sequence of bases beyond the stop signal, a portion of which serve as a signal to initiate addition of adenine residues (hereinafter poly A) 3' of the stop signal in the translated mRNA. The polyadenylation occurs mainly in the nucleus and is mediated by poly A polymerase that adds one adenylic acid residue at a time. Poly A does not code for an amino acid sequence after the stop codon has terminated translation, but is thought to contribute to stabilization of mRNA's and to the efficiency of translation of the mRNA coding region into amino acids.

The poly A in the mRNA of mammalian cells lies in a sequence known as the 3'untranslatable (hereinafter 3'UT) end portion. The 3'UT typically extends from the termination codon for the translation product and the terminus of the poly A. The 3'UT regions of mammalian mRNA's typically have an area of homology, known as the AAUAAA hexanucleotide sequence. This sequence is thought to be the poly A addition signal. It often precedes by 11 to 30 bases the poly A addition site.

The function, if any, of the 3'UT and poly A region has been investigated recently by Soreq et al. (Proc. Natl. Acad. Sci. U.S.A. Vol. 78, No. 3, pps. 1741 - 1745, March, 1981); Zaret et al. (J. Mol. Biol., 1984 Vol. 176, pp. 107-135); Baralle (International Review of Cytology, Vol. 81, pp. 71-106); and Ross et al. (J. Mol Biol., 1983, Vol. 167, pp. 607-617).

Soreq et al. reported that removal of the poly A and approximately 100 adjacent residues from a first human fibroblast beta interferon mRNA did not alter the translational activity or the functional stability of this mRNA in oocytes, whereas the deletion of the poly A and approximately 200 adjacent residues did decrease its translational efficiency. The removal of approximately 200 poly A residues and 200 adjacent residues from a second beta interferon mRNA did not alter either the translational activity or functional stability of the mRNA in oocytes. These authors concluded that neither the poly A residues nor large segments of the 3' noncoding region are required for the maintenance of the functional stability of human beta interferon mRNAs in such oocytes.

Zaret et al studied the cyc 1 - 512 mutant of the yeast S. cerevisiae which contains a 38 base pair deletion in the 3' noncoding region of the CYC-1 gene which encodes iso - 1 - cytochrome c. They reported that different 3' noncoding sequences which arose by chromosomal rearrangement increased the stability of CYC-1 mRNA and have varying effects upon the mRNA translational efficiency.

In Baralle's publication, The Functional Significance of Leader and Trailer Sequences in Eucaryotic mRNAs, the author reported that there is "no obvious function for the 3' non coding region" and that "the occurrence of sizable deletions or insertions during evolution of these genes suggests that the particular sequences which comprise the 3' non coding region are not essential to mRNA function." Baralle reported however that poly A does have a role in promoting mRNA stability. For instance, it has been found that removal of the poly A segment from globin mRNA greatly decreases the half-life of the mRNA in oocytes. However, Baralle suggests that poly A is apparently not necessary for successful translation as has been demonstrated by studies wherein the poly A has been removed from the mRNA.

Ross and Pizarro studied the hypothesis that steady state levels of human beta and delta globin proteins are determined in part by the intracellular stability of their respective messenger RNAs. They found that the rapid turnover of delta globin in mRNA accounts, at least in part, for the low level of delta globin mRNA in non nucleated peripheral blood reticulocytes, and speculated that the rate of mRNA decay may be determined by nucleotide sequence signals located in the 3' untranslated region. They observed that the 5' untranslated regions of beta and delta globin mRNAs are similar, but that their 3' untranslated regions differ significantly, and proposed that it should be possible to test the role of the 3' untranslated region in determining mRNA stability by comparing the half lives in transfected cells of chimeric mRNAs containing beta or delta 3' termini.

European Patent 0077689 discloses a method of gene manipulation wherein a yeast is transformed with a gene in which the 3'UT of a structural gene is added downstream from an exogenous gene. The transformant reveals a higher level of expression when the exogenous gene includes the 3'UT. In fact, studies revealed that the expression in the yeast carrying the plasmid with the region corresponding to the 3'UT as added is about 10-fold as compared with the yeast carrying the plasmid with no such region added.

EP 0 237 157 discloses a method of increasing levels of protein by use of an enhancer gene. EP 0 237 157 goes on to suggest that truncating the UT region can enhance the stability of the mRNA in combination with the combination gene.

EP 0 173 552 discloses the construction of a portable polyA addition signal from the bovine growth hormone gene, and the use of the portable polyA signal to increase production of TPA. The portable polyA signal is made by successive deletions on either side of the AATAAA sequence.

It is an object of this invention to provide a product and process for efficient production of a desired protein including human tPA in a myeloma cell line. Another object is to provide vectors for transfecting myeloma cells to induce high level expression of a gene encoding a desired protein. Another object is to provide transformants which when cultured produce large amounts of protein for therapeutic, diagnostic, and related uses. Still another object of the invention is to provide methods and recombinant native DNAs which promote mRNA stability in transfected myeloma cell lines by reducing the rate of intracellular mRNA decay, thereby increasing levels of expression.

These and other objects of the invention will be apparent to those skilled in the art from the following description and claims.

### Summary of the Invention

A process has now been discovered for increasing production of selected proteins in mammalian cell lines which normally secrete immunoglobulins. It has been found that a short segment of a 3'UT region and the polyadenylation region, if recombined 3' of the stop codon in a DNA coding for a protein of interest, are effective in promoting an increase in production of the protein of interest. The sequences comprising the small segment of 3'UT and the polyadenylation site appear to stabilize mRNA corresponding to the translated region and increase translation levels. The steady state level of the mRNA encoding the protein of interest is increased relative to mRNAs having longer untranslated regions.

In accordance with the invention, this discovery is exploited to provide vectors and processes for producing a protein of interest and to produce transformants which may be cultured to produce such materials at improved levels of expression. Either exogenous or endogenous proteins may be produced in accordance with the invention, i.e., one can produce proteins not encoded in the natural genome of the host cells, proteins which are encoded but not normally expressed in the host cells, or proteins which normally are expressed only at low levels.

In accordance with the invention, a DNA including a coding region, a stop signal codon, and a native 3'UT including a polyadenylation signal for the protein of interest is derived from a cell which naturally produces the protein of interest. The nucleotide sequence of the 3'UT region of this DNA is altered to produce an intact, transcriptionally competent, shorter recombinant native DNA having an altered 3'UT having less than about 300 nucleotide bases between the stop signal and the AATAAA polyadenylation signal. The recombinant DNA is transfected into a selected mammalian cell line which normally secretes immunoglobulins. The resulting transfected cell line is cultured to produce the protein of interest. The amount of protein produced is greater than that of the same cell line containing unaltered DNA for the protein of interest containing the longer native 3'UT.

In one embodiment, the shorter recombinant DNA contains a portion of the 3'UT of the DNA of the protein of interest.

In other embodiments, at least a portion of the altered 3'UT is obtained from a non-cellular DNA, e.g., viral DNA, while in others it is obtained from a mammalian DNA. The protein of interest may be an immunoglobulin or fraction thereof, a hormone, vaccine, lymphokine, cytokine, enzyme or pro-enzyme. For instance, the desired proteins may comprise a plasminogen activator such as human tissue plasminogen activator, the production of which is used as a model herein and discribed in detail. The mammalian cell line is a culturable cell line such as a myeloma cell line, though other mammalian cells which normally secrete immunoglobulins may be used.

In accordance with the invention, the process may comprise the additional step of combining proximate the DNA encoding the protein of interest, a DNA comprising a cellular enhancer element to enhance transcription of the recombinant DNA. The invention may further feature a blocking element as described in copending application Serial No. 837,595 filed March 7, 1986. Such blocking elements enable production of transformants which produce high levels of a desired protein while producing the marker protein only at levels required for selection.

The invention further features a vector for producing a protein when transfected into a mammalian cell line that normally secretes immunoglobulins. The vector is composed of transcriptionally competent DNA produced in accordance with the above summarized process. This vector improves production of a protein in a myeloma cell line relative to otherwise identical vectors encoding a protein of interest having a non-truncated untranslated region.

The invention further features a transformant for producing a protein of interest. The transformant preferably comprises a mammalian cell which normally produces immunoglobulins such as a myeloma cell. The transformant contains the vector harboring the expressable DNA summarized above. The transformant is capable of producing increased quantities of the protein of interest relative to otherwise identical transformants containing recombinant DNA including the full length 3' untranslated region native to the gene encoding the protein of interest

The enhanced expression levels produced in accordance with the invention are apparently caused by an increase in the steady state level of corresponding mRNA in transfected cells. It is believed that the shortness of the 3' UT region of the mRNA serves to reduce degradation of the mRNA within the cell, thereby increasing the intracellular half-life of the mRNA, and increasing expression levels.

It is known that phosphodiester bridges of DNA and RNA are attacked by exonucleases which act on either the 3' or 5' end of the molecule. Enzymes which act on the 3' end hydrolyze the ester linkage between the 3' carbon and the phosphoric group, while the 5' enzymes hydrolyze the ester linkage between the phosphoric group and the 5' carbon of the phosphodiester bridge. Endonucleases do not require a free terminal 3' or 5'-hydroxyl group; they attack 3' or 5' linkages wherever they occur in the polynucleotide chain.

It is hypothesized that recombinant DNAs comprising a long 3' UT are digested when an endonuclease cleaves a portion of the mRNA of the 3' UT not bound by a ribosome, followed by exonuclease digestion. Because of the shortness of the 3'UT region of the mRNA, when it associates with the ribosomes, less mRNA is left unbound to ribosomes and the number of sites for endonuclease attack are reduced. The presence of the poly A site protects the 3' end from exonucleases. The rate of degradation of mRNA is therefore decreased due to the shortness of the 3'UT region. Therefore, by the mechanism of the invention, mRNA is stabilized and its biological function increased to result in the increased expression of desired proteins.

For a fuller understanding of the nature and objects of the invention, reference should be made to the following detailed description and accompanying drawings.

### Brief Description of the Drawing

Fig. 1 is a schematic diagram illustrating the overall procedure of the invention;
Fig. 2 is a diagram illustrating various segments of DNA fused with a gene encoding a desired protein product (tPA); and
Fig. 3 is a diagram of plasmid pEMp-tPA, the preferred expression vector for use in the manufacture of tPA in myeloma cells. This vector represents the currently preferred embodiment of the invention.

### Description of the Invention

The invention provides a method, vector and transformant for increasing production of selected proteins in mammalian cells. The 3'UT region of a DNA encoding a protein of interest is altered to produce a shorter recombinant DNA having an 3'UT region, generally comprising less than about 300 nucleotide bases and may comprise less than 200 bases between the stop codon of the gene and the AATAAA poly A signal. A mammalian cell line which normally produces and secretes immunoglobulins is transfected with the resultant recombinant DNA. The transformant is cultured to produce increased amounts of the protein of interest as compared with the same cell line transfected with an unaltered 3'UT region.

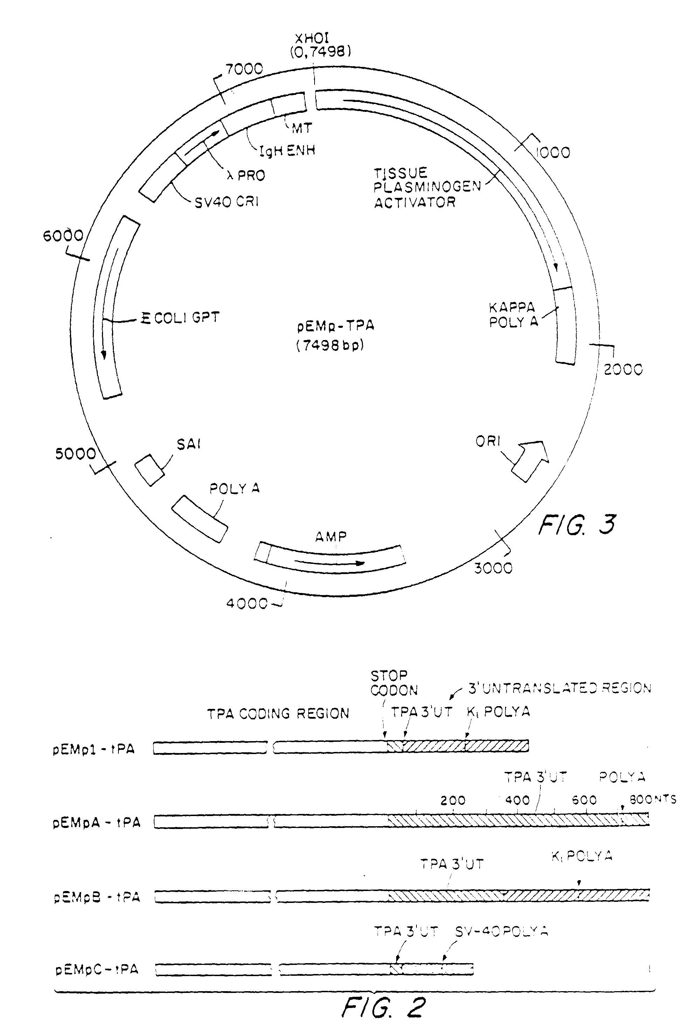

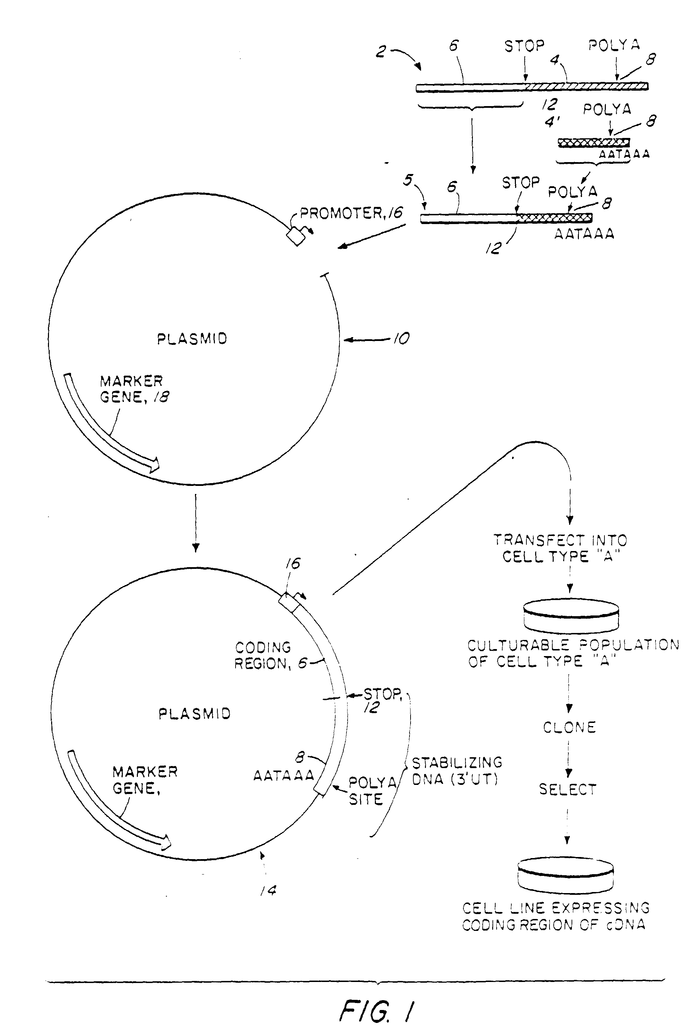

The process of the invention is represented in Figure 1. DNA 2 comprises a coding region 6 which encodes a protein of interest. It-is derived from a cell which naturally produces the protein, e.g., synthesized by conventional techniques with knowledge of the DNA sequence. For instance, the DNA may be a naturally occurring DNA or a chemically synthesized DNA. Alternatively, the DNA may be a reverse transcribed cDNA derived by conventional cDNA techniques. The typically long nucleotide sequence of the untranslated region 4 of this DNA 2 is altered to produce an intact, transcriptionally competent, shorter recombinant DNA 5 including the region 6 encoding the protein of interest, and beyond the stop signal; a 3' UT (7) less than about 300 nucleotide bases long linked to a polyadenylation addition signal 8. The recombinant DNA 5 therefore comprises a coding region for the protein of interest 6, a stop signal 12 and a shorter 3' UT designated 7 interposed between stop signal 12 and a poly A addition signal 8. The 3'UT including the Poly A site of the recombinant DNA may be obtained from the DNA 2 encoding the protein of interest by splicing out a segment of the 3' UT. The recombinant DNA 5 is inserted into a plasmid 10 to produce an expression vector 14. The plasmid includes a promoter sequence 16 and an expressable marker gene 18. A mammalian cell line is transfected with the recombinant DNA, cloned, and screened for a subpopulation of cells which have successfully incorporated and can express the gene 6. The transfected cell line is cultured to produce the desired protein which is then isolated and purified.

It has been noted that the 3'UTs of the highly abundant immunoglobulin mRNA's in lymphoid cells which secrete immunoglobulins are relatively short (less than about 300 nucleotides), whereas the 3'UTs of several genes of interest not expressed in lymphoid cells are relatively long. For example, the UT region of tPA is approximately 800 nucleotides long (Pennica et al, Nature, V. 301, 214-231, Jan., 10=983), that of Factor VIII about 1800 nucleotides (Wood et al, Nature, V. 312, 330-337, Nov., 1984), and that of erythropoietin about 560 nucleotides long (Jacobs et al, Nature, V. 313, 806-810, Feb., 1985).

The recombinant DNA techniques for manufacturing expression vectors and transformants useful in this invention are well-known and developed. They involve the use of various restriction enzymes which make sequence-specific cuts in DNA to produce blunt ends or cohesive ends, DNA ligases, techniques enabling enzymatic addition of sticky ends to blunt-ended DNA molecules, cDNA synthesis techniques, synthetic probes for isolating genes having a particular function, assembly of oligonucleotides to produce synthetic DNAs, conventional transfection techniques, and equally conventional techniques for cloning and subcloning DNA.

Various types of vectors may be used such as plasmids and viruses including animal viruses and phages. The vector will normally exploit a marker gene 18 which imparts to a successfully transfected cell a detectable phenotypic property which can be used to identify which individuals of a population of cells have successfully incorporated the recombinant DNA of the vector 14. Preferred markers for use in the myeloma cell lines comprise DNAs encoding an enzyme normally not expressed (or expressed only at low levels) by the cells which enable the cells to survive in an medium containing a toxin. Examples of such enzymes include thymidine kinase (TK), adenosine phosphoribosyltransferase (APRT), and hypoxanthine phosphoribosyl transferase (HPRT), which enable TK, APRT, or HPRT-deficient cells, respectively, to grow in hypoxanthine/aminopterin/thymidine medium; dihydrofolate reductase (DHFR), which enables DHRF-deficient cells to grow in the presence of methotrexate; the E.coli enzyme xanthine-guanosine phosphoribosyl transferase (XGPRT, the product of the gpt gene), which enables normal cells to grow in the presence of mycophenolic acid; and the procaryotic enzyme Tn5 phosphotransferase, which enables normal cells to grow in the presence of neomycin. Other suitable marker genes will be useful in the vectors used to transform myeloma cells in accordance with the invention.

Vectors of the invention may include an enhancer element of the type which acts on promoters disposed on both the 5' and 3' end of the enhancer element, or spaced apart from either end, to enhance transcription of genes located on the 3' end of the promotors. The enhancer element may include DNA of viral origin such as those disclosed by Banerji et al (Cell, V. 27, 299-308, 1981), deVilliers and Shaffner (Nucl. Acids Res., V. 9, 6251-6264, 1981), or Levinson et al. (Nature, V. 295, 568-572, 1982), but preferably is a cellular enhancer element of the type recently discovered by Gillies and Tonegawa and disclosed in Cell, (V. 33, 717-728, 1983), and in more detail in copending U.S. Application Serial No. 592,231, filed March 22, 1984. The vector may further include a blocking element as described in copending application Serial No. 837,595 filed March 7, 1986, which enables production of transformants which produce high levels of a desired protein under the influence of the enhancer while producing the marker protein only at lower levels required for selection.

Figure 3 represents the restriction map of the preferred plasmid vector used for the production of tPA in accordance with the invention. This vector is designated pEMp-tPA and comprises 7498 base pairs. It includes a myeloma cell-derived enhancer element which enhances transcription of mRNA from inserted DNA encoding protein, here human tPA (see Gillies et al. Cell, 1983, supra), and exploits vector construction principles which regulate selectively the enhancer function in accordance with the disclosure of copending U.S. application Serial No. 837,595. Details of the vector's constructions are disclosed below.

In preferred embodiments, the host cell system to be transfected with the recombinant DNA is a continuous or established cell line which has undergone a change enabling the cells to grow indefinitely. These cells therefore are unlike normal cells that divide in culture for a limited number of generations before senescing. The ability to grow indefinitely further assists in scaling-up production of proteins of interest.

The host cells used for the practice of the invention are mammalian cells that normally produce and secrete immunoglobulins. These cells normally reside in the circulatory system. The cell actually used may have lost the ability to secrete immunoglobulins through previous selection.

It is further preferred that the host system comprise murine or rat derived cells (as opposed to human cells) to reduce the possibility of contamination of product protein by human viruses. In preferred embodiments, myeloma cells constitute the host culture system. Myeloma cells are typically easily cultured and secrete expression products into the extracellular medium.

The vector disclosed in Figure 3, and other suitable expression vectors, are used in accordance with the invention to transfect myeloma cells, preferably of murine origin, such as cell line J558 (ATCC-TIB 6), Sp2/0-Ag14 (ATCC CRL 1581), and P3X63-Ag8.653 (ATCC CRL 1580). The preferred myeloma cell line is J558L (ATCC CRL 9132, See Oi et al. Proc. Natl. Acad. Sci., USA, V. 80, p. 825 1983). These cells comprise malignant transformants of B cells from the circulatory system of the Balb/C mouse, and are derived from myeloid tissue.

The transformants of the cells of the type set forth above may be cultured in suspension or preferably within microcapsules in accordance with the procedure disclosed in U.S. Patent No. 4,409,331 to F. Lim. For ease of purification, and to promote post-secretion stability of the protein product, it is preferred that the cells be cultured in serum-free medium. The extracellular protein is harvested daily as medium is replaced. This approach has the advantage of producing the product free of contaminating proteolytic enzymes and other inactivating factors often present in bovine or equine sera. The preferred J558L cell secretes lambda light chain of immunoglobulin A in significant quantities. This and other contaminating proteins can be separated readily from the protein product.

The invention may be used to manufacture large amounts of valuable proteins in additions to tPA in cells genetically engineered as disclosed herein. Non-limiting examples include other plasminogen activators and proactivators, blood clotting factors, hormones, interferons, antibodies, enzymes and pro-enzymes, vaccines, and various lymphokines and cytokines.

The example which follows should be understood to be exemplary in all respects, providing a detailed disclosure of a tPA production protocol embodying the invention, and a disclosure of the best mode currently known of practicing the invention.

### Example -

### The Production of tPA-Producing Myeloma Transformants

The basic expression vector, designated pEMpl, was constructed from the following fragments: (a) a 2.25 PvuII-BamHI fragment from pSV2-gpt (Mulligan and Berg, Science; V. 209, 1422-1427, 1980) containing the SV40 enhancer and early region promoter, the E. coli gpt gene, the SV40 small tumor-antigen intervening sequence, and the SV40 transcription termination and polyadenylation signals; (b) a 2.3 kb pVuII-EcoRI fragment from pBR322 containing the ampicillinase gene and the bacterial origin of replication (Sutcliffe, Proc. Natl. Acad. Sci., USA, V. 75, 3737, 1978); (c) a 0.3 kb PvuII-EcoRI fragment containing an immunoglobulin heavy chain enhancer (Gillies et al, Cell, V. 33, p. 717-728, 1983); (d) a 0.25 kb SacI-BglII fragment containing the metallothioneine I promoter (Brinster et al, Nature, V. 296, 39-42, 1982); and (e) a 0.4 kb AvaII-HaeIII fragment from the 3'UT region of the immunoglobulin kappa light chain gene, which includes the polyadenylation signal (Max et al, J. Biol. Chem., V. 256, 5116-5120, 1981). These fragments were ligated together in a series of reactions according to well known methodologies (see, e.g., Maniatis et al, Molecular Cloning: A Laboratory Manual, Cold Spring Harbor, 1982).

The basic expression vector was used to construct a series of vectors expressing tPA. Designated pEM1-tPA-A, -B, and -C, these three vectors all contained the entire coding region of the tPA cDNA, but differed in their 3' untranslated regions as described below.

The cDNA for tPA was obtained by standard techniques (Maniatis, supra). TPA clones containing the entire coding region, and approximately 800 base pairs of the 3' UT region were identified and confirmed by nucleotide sequencing. The tPA cDNA was truncated at the Bgl II site approximately 400 base pairs downstream of the translation stop codon and inserted via Xho I linkers into the unique XhoI site in the basic vector. The tPA 3" UT region in this expression vector, pEM1-tPA-A, therefore was composed of approximately 400 base pairs of the native tPA 3' UT and 200 base pairs of the 3" UT region of the immunoglobulin kappa light chain gene.

In vector pEM1-tPA-B the bulk of the native tPA 3' UT was eliminated by cloning at the Sau 3A site located 34 base pairs downstream of the translation stop signal and joining to the 200 base pair 3' UT of the immunoglobulin kappa light chain gene.

Vector pEM1-tPA-C differed from pEM1-tPA-B in that the 3' UT region of the kappa light chain gene was replaced with an approximately 200 base pair fragment from the 3' UT region of the SV40 late genes.

The recombinant tPA genes with the various 3' UT regions described above are depicted in Fig. 2. There is about .59 kb between the stop codon and the AATAAA polyaddition signal in vector pEMl-tPA-A, .23 kb in pEM1-tPA-B, and .16 kb in pEM1-tPA-C.

The tPA-containing plasmids were transfected into J558L myeloma cells by the protoplast fusion method (Gillies et al, supra). Cells containing the plasmid, and therefore the gpt gene, were selected by culturing in media containing mycophenolic acid. Resistant colonies containing the plasmids were subcloned and screened for tPA expression. The synthesis and secretion of tPA into the medium was determined by means of an assay in which tPA converts plasminogen to plasmin, which then cleaves the chromogenic tripeptide S2251 (Pennica et al, supra). Because it is known that serum contains inhibitors of both tPA and plasmin (Collen and Lijnen, CRC Critical Reviews in Oncology/Hematology, V. 4, 249-301, 1986), transformants were cultures for 48 hours in serum-free medium before assay. Activity was measured in international units (IU) based on a standard supplied by the world Health Organization and confirmed by a tPA standard obtained from American Diagnostics, Inc.

No cloning expressing significant quantities of tPA could be found in the cells transfected with vector pEM1-tPA-A, although analysis indicated the presence of low levels of mRNA encoding tPA. Of 26 clones transfected with pEM1-tPA-B, 5 expressed tPA. If the highest expression level is given a relative value of 1.00, the expression of tPA by pEM1-tPA-B transfectants ranged between 0.28 and 1.00. Of 14 clones transfected with pEMl-tPA-C, 6 expressed tPA at a relative level of 0.12-0.44.

The invention may be embodied in other specific forms without departing from the spirit and scope thereof.

## Claims

1. A process for improving the production of a protein in a myeloma cell line by enhancing the stability of mRNA encoding the protein, said process comprising the steps of:
a) providing DNA derived from a cell which naturally produces said protein, said DNA comprising in sequence a coding region, a stop signal codon, and a native untranslated region including a polyadenylation signal, the DNA optionally further comprising a cellular enhancer element proximate to said coding region to enhance transcription;
b) reducing the number of bases of said native 3' untranslated region of said DNA by splicing out a segment of the 3' untranslated region to produce an intact, transcriptionally competent, shorter DNA having an altered untranslated region comprised wholly of native DNA and having less than about 300 nucleotide bases between said stop signal codon and a polyadenylation signal;
c) transfecting said myeloma cell line with said shorter DNA; and
d) culturing said transfected cell line to produce said protein, the amount of protein produced by said transfected cell line being greater than the amount of protein produced by an otherwise identical cell line containing the DNA described in step (a).

2. A process for improving the production of a protein in a myeloma cell line by enhancing the stability of mRNA encoding the protein, said process comprising the steps of:
a) providing a DNA derived from a cell which naturally produces said protein, said DNA comprising in sequence a coding region, a stop signal codon, and a native untranslated region including a polyadenylation signal, the DNA lacking an enhancer sequence;
b) altering the nucleotide sequence of said native 3' untranslated region of said DNA to produce an intact, transcriptionally competent, shorter DNA having an altered untranslated region comprising less than about 300 nucleotide bases between said stop signal codon and a polyadenylation signal;
c) transfecting said myeloma cell line with said shorter DNA which lacks an enhancer sequence; and
d) culturing said transfected cell line to produce said protein, the amount of protein produced by said transfected cell line being greater than the amount of protein produced by an otherwise identical cell line containing the unaltered DNA described in step (a) in the absence of an enhancer sequence.

3. The process of either claim 1 or claim 2, wherein said altered untranslated region includes the sequence AATAAA, where A is adenine and T is thymine.

4. The process of claim 2 and claims dependent thereon, wherein step (b) further comprises ligating to said shorter untranslated region a 3' untranslated sequence non-native to said DNA to produce a hybrid 3' untranslated region between said stop signal codon and the polyadenylation signal.

5. The process of claim 4, wherein at least a portion of said non-native untranslated region is obtained from non-mammalian DNA or from mammalian DNA, e.g. from the 3'UT of an immunoglobulin mRNA.

6. The process of any one of the preceding claims, wherein said coding region encodes a protein selected from immunoglobulins, hormones, vaccines, lymphokines, cytokines, enzymes and pro-enzymes, for example a plasminogen activator e.g. human tissue plasminogen activator.

7. A vector for improving the production of a protein in a myeloma cell line, said vector comprising replicable, transcriptionally competent DNA having in sequence a first DNA segment comprising a promoter sequence, a second DNA segment encoding said protein, a stop signal codon, and a third DNA segment 3' of said stop signal codon comprising a polyadenylation signal and a truncated untranslated region comprised wholly of DNA native to the gene encoding said protein and having less than about 300 base pairs between said stop codon and the polyadenylation signal, said vector having optionally a cellular enhancer element proximate to said DNA encoding said protein and being operable to produce increased quantities of protein relative to otherwise identical vectors comprising the 3' untranslated region native to the gene encoding said protein.

## Patentansprüche

1. Verfahren zur Verbesserung der Produktion eines Proteins in einer Myelom-Zellinie durch Erhöhen der Stabilität der für das Protein codierenden mRNA, wobei das Verfahren die folgenden Stufen umfaßt:
a) Bereitstellung von DNA, die aus einer Zelle stammt, die das Protein von Natur aus produziert, wobei die DNA in Sequenz einen codierenden Bereich, ein Stopcodon und einen nativen untranslatierten Bereich, der ein Polyadenylierungssignal enthält, umfaßt, und wobei die DNA außerdem gegebenenfalls ein nahe am codierenden Bereich liegendes zelluläres Enhancer-Element zur Verstärkung der Transkription enthält;
b) Verminderung der Anzahl der Basen dieses nativen 3'-untranslatierten Bereiches der DNA durch Spleißen eines Segmentes aus dem 3'-untranslatierten Bereich, um eine intakte transkriptionskompetente kürzere DNA mit einem veränderten untranslatierten Bereich, der vollständig aus nativer DNA besteht und weniger als etwa 300 Nucleotidbasen zwischen dem Stopcodon und einem Polyadenylierungssignal aufweist, zu erzeugen;
c) Transfektion der Myelom-Zellinie mit der kürzeren DNA; und
d) Kultivierung der transfizierten Zellinie, um das Protein zu produzieren, wobei die Menge des durch die transfizierte Zellinie produzierten Proteins größer ist als die durch eine sonst identische Zellinie, die die in Schritt (a) beschriebene DNA enthält, produzierte Menge des Proteins.

2. Verfahren zur Verbesserung der Produktion eines Proteins in einer Myelom-Zellinie durch Erhöhen der Stabilität der für das Protein codierenden mRNA, wobei das Verfahren die folgenden Schritte umfaßt:
a) Bereitstellung von DNA, die aus einer Zelle stammt, die das Protein von Natur aus produziert, wobei die DNA in Sequenz einen codierenden Bereich, ein Stopcodon und einen nativen untranslatierten Bereich, der ein Polyadenylierungssignal enthält, umfaßt, wobei der DNA eine Enhancer-Sequenz fehlt;
b) Änderung der Nucleotidsequenz des nativen 3'-untranslatierten Bereiches der DNA, um eine intakte, transkriptionskompetente kürzere DNA mit einem veränderten untranslatierten Bereich herzustellen, die weniger als etwa 300 Nucleotidbasen zwischen dem Stopcodon und einem Polyadenylierungssignal umfaßt;
c) Transfektion der Myelom-Zellinie mit der kürzeren DNA, der eine Enhancer-Sequenz fehlt; und
d) Kultivierung der transfizierten Zellinie, um das Protein zu erzeugen, wobei die Menge des durch die transfizierte Zellinie produzierten Proteins größer ist als die durch eine sonst identische Zellinie, die die in Schritt (a) beschriebene DNA ohne eine Enhancer-Sequenz enthält, produzierte Menge des Proteins.

3. Verfahren nach entweder Anspruch 1 oder Anspruch 2, wobei der veränderte untranslatierte Bereich die Sequenz AATAAA enthält, worin A Adenin und T Thymin bedeutet.

4. Verfahren nach Anspruch 2 und nach dessen Unteransprüchen, wobei Stufe (b) außerdem folgendes umfaßt: Verknüpfen einer 3'-untranslatierten, zu der DNA nicht nativen Sequenz mit dem kürzeren untranslatierten Bereich, um einen 3'-untranslatierten Hybridbereich zwischen dem Stopcodon und dem Polyadenylierungssignal zu erzeugen.

5. Verfahren nach Anspruch 4, wobei wenigstens ein Teil des nicht nativen, untranslatierten Bereichs aus Nichtsäuger-DNA oder aus Säuger-DNA, z.B. aus der 3'-UT einer Immunglobulin-mRNA, erhalten wird.

6. Verfahren nach einem der vorhergehenden Ansprüche, wobei der codierende Bereich für ein Protein, ausgewählt aus Immunglobulinen, Hormonen, Impfstoffen, Lymphokinen, Cytokinen, Enzymen und Proenzymen, beispielsweise für einen Plasminogenaktivator, z.B. für einen Plasminogenaktivator aus menschlichem Körpergewebe, codiert.

7. Vektor zur Verbesserung der Produktion eines Proteins in einer Myelom-Zellinie, wobei der Vektor replizierbare transkriptionskompetente DNA enthält, die in Sequenz folgendes aufweist: ein erstes DNA-Segment, das eine Promotor-Sequenz enthält, ein zweites DNA-Segment, das für das Protein codiert, ein Stopcodon und ein drittes DNA-Segment 3' zu dem Stopcodon, das ein Polyadenylierungssignal, und einen verkürzten untranslatierten Bereich enthält, der vollständig aus DNA besteht, die zu dem für das Protein codierende Gen nativ ist und weniger als etwa 300 Basenpaare zwischen dem Stopcodon und dem Polyadenylierungssignal aufweist, wobei der Vektor gegebenenfalls ein zelluläres, nahe an der für das Protein codierenden DNA liegendes Enhancer-Element aufweist und geeignet ist, relativ zu sonst identischen Vektoren, die den 3'-untranslatierten, zu dem für das Protein codierenden Gen nativen Bereich enthalten, vergrößerte Mengen des Proteins zu produzieren.

## Revendications

1. Procédé pour améliorer la production d'une protéine dans une lignée cellulaire de myélome, par augmentation de la stabilité de l'ARNm codant pour la protéine, ledit procédé comprenant les étapes consistant à :
a) fournir de l'ADN dérivé d'une cellule qui produit naturellement ladite protéine, ledit ADN comprenant dans sa séquence une région codante, un codon signal stop, et une région native non traduite incluant un signal de polyadénylation, l'ADN comprenant en outre éventuellement un élément enhancer cellulaire à proximité de ladite région codante pour augmenter la transcription ;
b) réduire le nombre de bases de ladite région native 3' non traduite dudit ADN en épissant un segment de la région 3' non traduite pour produire un ADN plus court transcriptionnellement compétent et intact, ayant une région non traduite modifiée constituée intégralement d'ADN natif et ayant moins d'environ 300 bases nucléotidiques entre ledit codon signal stop et un signal de polyadénylation ;
c) transfecter ladite lignée cellulaire de myélome avec ledit ADN plus court ; et
d) cultiver ladite lignée cellulaire transfectée pour produire ladite protéine, la quantité de protéine produite par ladite lignée cellulaire transfectée étant plus grande que la quantité de protéine produite par une lignée cellulaire similaire contenant l'ADN décrit dans l'étape (a).

2. Procédé pour améliorer la production d'une protéine dans une lignée cellulaire de myélome, par augmentation de la stabilité de l'ARNm codant pour la protéine, ledit procédé comprenant les étapes consistant à :
a) fournir de l'ADN dérivé d'une cellule qui produit naturellement ladite protéine, ledit ADN comprenant dans sa séquence une région codante, un codon signal stop, et une région native non traduite incluant un signal de polyadénylation, l'ADN n'ayant pas de séquence enhancer ;
b) modifier la séquence nucléotidique de ladite région native 3' non traduite dudit ADN pour produire un ADN plus court transcriptionnellement compétent et intact ayant une région non traduite modifiée comprenant moins d'environ 300 bases nucléotidiques entre ledit codon signal stop et un signal de polyadénylation ;
c) transfecter ladite lignée cellulaire de myélome avec ledit ADN plus court à qui il manque une séquence enhancer ; et
d) cultiver ladite lignée cellulaire transfectée pour produire ladite protéine, la quantité de protéine produite par ladite lignée cellulaire transfectée étant plus grande que la quantité de protéine produite par une lignée cellulaire similaire contenant l'ADN non modifié décrit dans l'étape (a) en l'absence d'une séquence enhancer.

3. Procédé selon la revendication 1 ou la revendication 2, dans lequel ladite région non traduite modifiée inclut la séquence AATAAA, où A est l'adénine et T est la thymine.

4. Procédé selon la revendication 2 et les revendications dépendantes de celle-ci, dans lequel l'étape (b) comprend en outre la ligature, à ladite région non traduite plus courte, d'une séquence 3' non traduite non native par rapport audit ADN pour produire une région hybride 3' non traduite entre ledit codon signal stop et le signal de polyadénylation.

5. Procédé selon la revendication 4, dans lequel au moins une portion de ladite région non traduite non native est obtenue à partir d'ADN de non-mammifère ou d'ADN de mammifère, par exemple de la région 3'UT d'un ARNm d'immunoglobuline.

6. Procédé selon l'une quelconque des revendications précédentes dans lequel ladite région codante code pour une protéine choisie parmi des immunoglobulines, des hormones, des vaccins, des lymphokines, des cytokines, des enzymes et des pro-enzymes, par exemple un activateur de plasminogène tel qu'un activateur tissulaire de plasminogène humain.

7. Vecteur pour améliorer la production d'une protéine dans une lignée cellulaire de myélome, ledit vecteur comprenant de l'ADN réplicable, transcriptionnellement compétent ayant dans sa séquence un premier segment d'ADN comprenant une séquence promoteur, un second segment d'ADN codant pour ladite protéine, un codon signal stop, et un troisième segment d'ADN en 3' dudit codon signal stop comprenant un signal de polyadénylation et une région non traduite tronquée constituée intégralement d'ADN natif du gène codant pour ladite protéine et ayant moins d'environ 300 paires de base entre ledit codon stop et ledit signal de polyadénylation, ledit vecteur ayant éventuellement un élément enhancer cellulaire à proximité dudit ADN codant pour ladite protéine et étant capable de produire des quantités accrues de protéine par rapport à des vecteurs similaires comprenant la région 3' non traduite par rapport au gène codant pour ladite protéine.
